# FASCICULE DE BREVET EUROPEEN

(11) **EP 1 885 418 B1**
(45) Date de publication et mention de la délivrance du brevet: **02.11.2011**
(21) Numéro de dépôt: 06726242.8
(22) Date de dépôt: 13.03.2006
(51) Int. Cl.: A61M 5/44

(54) **TUBE IMPLANTABLE DESTINÉ À L'INJECTION NOTAMMENT DE FLUIDE CALOPORTEUR DANS TOUT OU PARTIE D'UN TISSU HUMAIN OU ANIMAL**
IMPLANTIERBARES RÖHRCHEN ZUM INJIZIEREN EINER WÄRMEÜBERTRAGUNGSFLÜSSIGKEIT IM GESAMMTEN ODER IN TEILEN DES MENSCHLICHEN ODER TIERISCHEN GEWEBES
IMPLANTABLE TUBE FOR INJECTION PARTICULARLY OF HEAT TRANSFER FLUID INTO ALL OR PART OF A HUMAN OR ANIMAL TISSUE

(30) Priorité: 12.04.2005 FR 0550936
(43) Date de publication de la demande: 13.02.2008
(73) Titulaire: CENTRE D'ETUDE ET DE RECHERCHE MEDICALE D'ARCHAMPS, 74160 Archamps (FR)
(72) Inventeur: MEHIER, Henri, 69002 Lyon (FR)
(74) Mandataire: Fleurance, Raphaël
(86) Numéro de dépôt international: PCT/FR2006/050218
(87) Numéro de publication internationale: WO 2006/108974

(56) Documents cités:
- WO-A-02/069821
- WO-A-03/070302
- FR-A- 2 790 965
- US-B1- 6 328 735
- E-MÉMOIRES DE L'ACADÉMIE NATIONALE DE CHIRURGIE, [Online] 26 mai 2004 (2004-05-26), XP002335290 2004 Extrait de l'Internet: URL:http://www.bium.univ-paris5.fr/acad-ch irurgie/ememoires/005_2004_3_2_43x50.pdf>

## Description

L'invention a pour objet un tube implantable destiné à l'injection de fluide dans tout ou partie d'un tissu humain ou animal. Dans la suite de la description, l'invention est plus particulièrement décrite en relation avec l'injection de fluide caloporteur. Néanmoins, le tube peut être également utilisé pour l'injection de substances froides telles que par exemple des suspensions de nanoparticules magnétiques ou de nanocapsules contenant une ou plusieurs matières actives.

Une des méthodes de traitement des tumeurs cancéreuses consiste à détruire en tout ou partie le tissu cancéreux par l'administration ciblée de chaleur ou de froid. Ce principe est connu sous la dénomination « thermoablation » et est actuellement mis en oeuvre notamment pour le traitement des métastases hépatiques.

Plusieurs techniques s'appuyant sur le principe de la thermoablation par la chaleur sont aujourd'hui proposées, telles que laser, radiofréquence avec aiguille, la cryothérapie relevant quant à elle de la thermoablation par le froid. Toutefois, ces techniques présentent un certain nombre d'inconvénients. En particulier, le volume de la tumeur traitée reste limité (en pratique de 4 à 5 cm de diamètre) et le temps d'intervention relativement long, de 20 à 30 minutes pour la radiofréquence et la cryothérapie, davantage encore pour le traitement au laser.

Le document WO 00/29055 du Demandeur décrit une technique de thermoablation par la chaleur consistant à injecter directement dans l'organisme de l'eau ou de l'eau oxygénée à une pression pouvant aller jusqu'à 3000 bars, à une température de 200 à 400°C. Pour ce faire, l'eau oxygénée ou l'eau est chauffée dans une bobine métallique incorporant une résistance électrique ou un échangeur de température autour de laquelle est entouré un tube platine-irridium. Le tube en alliage est raccordé au moyen de diffusion, lequel se présente sous la forme d'un tube implanté directement dans le tissu à traiter. Ce tube, désigné par la suite « microtube », a un diamètre externe compris entre 100 et 250 µm, et un diamètre interne compris entre 50 et 150 µm. Il est réalisé en un matériau apte à supporter la pression de 3000 bars tel que par exemple un alliage platine/irridium, et permet donc, lorsqu'il est connecté à la bobine de chauffage, d'injecter de l'eau ou de l'eau oxygénée sous forme vaporisée. La température du liquide vaporisé, au contact du tissu à traiter, diminue et l'eau redevient liquide au sein même de la tumeur.

Dans le document WO 03/070302, le Demandeur propose une méthode de thermoablation perfectionnée en ce qu'elle prévoit d'injecter le liquide caloporteur non plus en continu, mais sous forme pulsée. En pratique, le volume de liquide injecté est très faible, par exemple compris entre 0,2 et 1 ml ce qui permet d'éviter la diffusion de chaleur en dehors de la tumeur. En outre, ces volumes sont injectés à intervalles réguliers compris entre 0,5 et 1 seconde, ce qui permet de réduire la quantité de chaleur en dehors de la zone à traiter, facilitant ainsi la manipulation du tube par le chirurgien.

Dans les deux procédés proposés, le système de chauffage reste inchangé et consiste pour l'essentiel en une bobine métallique incorporant une résistance électrique et autour de laquelle est entouré un tube inox dans lequel circule le fluide caloporteur.

Ce système de chauffage présente un certain nombre d'inconvénients.

Tout d'abord, sa localisation en amont de l'installation nécessite une puissance de chauffage d'autant plus importante que la rallonge séparant le microtube en tant que tel du système de chauffage, est longue. En outre, ce système ne permet pas d'enchaîner, sans aucun temps mort, l'injection par pulses, de produits chauds et de produits froids, car le temps de refroidissement de la bobine est trop long.

Le document US-A-5,542,928 décrit un cathéter destiné à être mis en oeuvre pour la thermoablation dans lequel circule un fluide débouchant à l'extérieur au niveau de l'extrémité distale dudit tube par le biais de perforations. En pratique, le chauffage du liquide circulant dans le tube est obtenu au moyen d'une résistance hélicoïdale agencée sur la portion distale du cathéter et connectée à une source électrique. Le cathéter décrit dans ce document est destiné à être introduit dans des cavités pour lesquelles il n'est pas nécessaire d'avoir un tube de faible diamètre, le diamètre étant en effet compris entre 2 et 10 mm. La présence de la résistance augmente davantage encore le diamètre final du dispositif destiné à être introduit dans l'organisme. Si ce document décrit l'idée de chauffer uniquement la partie distale du tube, la taille du système proposé reste incompatible avec une implantation directement dans les tissus.

Le document US-6,328,735 B1 décrit une technique de thermoablation combinant injection de liquide chaud et radiofréquence. Plus précisément, de même que précédemment, l'installation décrite comprend un tube dont l'extrémité distale est munie d'une résistance destinée à chauffer le liquide arrivant à l'extrémité du tube. Là encore, le diamètre du tube est avantageusement de 2 mm et entouré d'une bobine de résistance égale à 50 Ω.

Le document US-5,964,752 décrit un appareil du même type que précédemment, destiné au traitement des cartilages. Là encore, l'extrémité distale du tube est munie d'une résistance, cette fois positionnée à l'intérieur du tube.

Dans tous les cas, les systèmes de chauffage proposés requièrent une résistance sous forme de bobine engendrant une inductance élevée incompatible avec les pulses de courant tels que ceux mis en oeuvre par le Demandeur, car cela occasionnerait une impédance trop élevée.

Le document WO 02/069821 décrit un tube dans lequel circule de la vapeur, destiné à être implanté dans l'organisme. La vapeur est générée directement dans le tube dans lequel circule le fluide par radiofréquence. Plus précisément, le tube présente deux électrodes connectées à un générateur de radiofréquences, le liquide assurant le passage du courant entre les deux électrodes. Dans le système proposé, il n'y a pas de chauffage différentiel et le tube est chauffé sur toute sa longueur. En outre, le choix du liquide à injecter est limité aux liquides conducteurs. Enfin, le passage du courant dans le liquide est susceptible d'affecter les propriétés de celui-ci.

En d'autres termes, le problème que se propose de résoudre l'invention est de développer une installation du type de celle décrite par exemple dans le document WO 03/070302, dans laquelle le fluide caloporteur est chauffé à la température de vaporisation, exclusivement au niveau de la partie distale du microtube implantable, le diamètre du tube étant de 10 à 20 fois plus petit que celui d'un cathéter utilisé traditionnellement pour la thermoablation.

Un second problème que se propose de résoudre l'invention est de développer un système dans lequel l'inductance soit nulle, de sorte à pouvoir être utilisé en pulses de courant.

Un troisième problème que se propose de résoudre l'invention est de fournir une installation unique qui permette l'injection de fluide caloporteur ou de fluide froid véhiculant des nanocapsules ou des nanoparticules, en fonction de la nature du traitement.

Le Demandeur a développé un microtube connecté à une source électrique présentant une structure telle qu'il a une résistance comprise entre 0,2 et 2 Ω dans sa partie distale et une résistance inférieure à 0,01 Ω dans la partie restante.

En d'autres termes, la puissance électrique est concentrée dans la partie distale du tube, permettant ainsi d'atteindre des températures de l'ordre de 400°C.

Le nouveau système développé consiste en un microtube destiné à être implanté directement dans des tissus, le microtube étant dépourvu de résistance électrique additionnelle et présentant une extrémité distale apte à atteindre des températures de chauffage élevées, le tube lui-même faisant office de résistance chauffante. Ce tube est relié par le biais d'une rallonge dans laquelle circule le liquide froid, à l'unité de stockage et d'injection de liquide.

En d'autres termes, l'invention a pour objet un tube implantable apte à chauffer par conduction, notamment un fluide caloporteur destiné à être injecté dans tout ou partie d'un tissu humain ou animal, le tube étant muni d'une paroi présentant des parties distale, médiane et proximale et comprenant des moyens de solidarisation directs ou indirects de la partie proximale à un réservoir de fluide, le tube se caractérisant en ce qu'il est muni de deux moyens de connexion aux bornes d'une source électrique assurant l'arrivée et le retour du courant dans la paroi, et en ce qu'il présente une résistance inférieure à 0.01 Ω dans ses parties médiane et proximale et une résistance comprise entre 0.2 et 2 Ω dans sa partie distale, les parties médiane et proximale étant reliées électriquement en série à la partie distale, le tube étant dépourvu de résistance électrique additionnelle et présentant une inductance pratiquement nulle.

L'objectif poursuivi est donc de disposer d'un microtube qui puisse être implanté dans l'organisme, et dans lequel le liquide caloporteur soit chauffé électriquement et par conduction, uniquement au niveau de la partie distale du microtube, c'est-à-dire dans la zone à traiter et non dans les parties proximale et médiane, permettant ainsi d'éviter de chauffer les zones de tissu connexes et de faciliter la manipulation de l'extrémité proximale libre du tube par le chirurgien.

Pour disposer d'un microtube présentant les résistances précitées, la paroi du tube implantable présente dans un mode de réalisation avantageux, du centre vers sa périphérie, en section :
- un tube métallique dans lequel circule le fluide à injecter,
- à l'exception de la partie distale, une gaine assurant l'arrivée du courant réalisée en un matériau conducteur solidaire d'un moyen de connexion à une des bornes de la source électrique,
- à l'exception de l'extrémité libre de la partie distale, une gaine isolante électriquement,
- une gaine assurant le retour du courant réalisée en un matériau conducteur solidaire d'un moyen de connexion à l'autre borne de la source électrique.

Avantageusement, la paroi du tube est munie d'une gaine extérieure biocompatible permettant son implantation dans les tissus.

Selon une première caractéristique, le tube métallique a une résistivité comprise entre 20 et 100 µΩ.cm et présente avantageusement une longueur inférieure à 50 cm. En pratique, le diamètre externe du tube est compris entre 200 µm et 800 µm, avantageusement égal à 250 µm, de même que le diamètre interne est compris entre 100 µm et 250 µm, avantageusement égal à 150 µm.

En pratique, le tube métallique est réalisé en un matériau du type alliage platine/irridium, titane, acier inoxydable, alliage nickel-titane et plus généralement, tout matériau conducteur électrique apte à supporter une pression jusqu'à 3000 bars et une température de 400°C.

Pour assurer l'arrivée et le retour du courant, la gaine est constituée d'un revêtement électrolytique de cuivre d'épaisseur comprise entre 20 µm et 50 µm.

En ce qui concerne la gaine isolante électriquement, celle-ci est avantageusement constituée d'un revêtement sous vide d'oxyde de titane ou d'alumine et plus généralement tout matériau isolant électrique et résistant à une température d'au moins 400°C. L'épaisseur de la gaine est comprise entre 200 nm et 400 nm.

De même, la gaine extérieure est de préférence réalisée en un revêtement électrolytique d'or d'épaisseur d'environ 1 µm.

Le microtube de l'invention peut être utilisé de deux manières différentes.

Tout d'abord, il peut être implanté durablement dans les tissus pour un traitement au long cours. Dans cette hypothèse, la partie distale du tube implantable est munie de perforations de taille comprise entre 50 µm et 150 µm, avantageusement égale à 70 µm, tandis que son extrémité libre est obturée. En pratique, le microtube est implanté au moyen d'une aiguille fendue, ladite aiguille étant ensuite retirée pour ne laisser dans le tissu, que le tube.

Le traitement peut être aussi réalisé de manière ponctuelle. Dans une telle hypothèse, le microtube est retiré immédiatement après utilisation. Il est dépourvu de perforations latérales dans sa partie distale et présente un simple orifice débouchant, ménagé à son extrémité distale libre. Dans ce cas, le microtube est utilisé en combinaison avec une aiguille dont les parois sont munies de perforations, le microtube étant introduit dans le corps de l'aiguille et le tout implanté dans le tissu à traiter.

L'invention concerne également une installation destinée notamment à l'injection de fluide caloporteur en régime pulsé dans tout ou partie d'un tissu humain ou animal, mettant en oeuvre le microtube précédemment décrit.

Plus précisément, une telle installation contient :
■ une unité de stockage du fluide ;
■ une unité d'injection dudit fluide ;
■ le tube implantable précédemment décrit ;
■ une rallonge reliant l'extrémité proximale du tube implantable à l'unité d'injection.

L'unité d'injection se présente en pratique sous forme d'une chambre contenant la substance à injecter et dans laquelle un vérin hydraulique de faible diamètre, de l'ordre de 3 à 5 mm, est piloté par un vérin électrique, pneumatique, piézo-électrique ou mécanique, de diamètre plus important, de l'ordre de 50 à 80 mm, dont le déclenchement et/ou la course et/ou la force et/ou la vitesse de déplacement sont déterminés en fonction du rythme, du volume et de la pression d'injection souhaitée de la substance dans la rallonge par le vérin hydraulique.

Pour éviter le retour de la substance dans la rallonge après injection de ladite substance, l'unité d'injection contient deux clapets anti-retour. Comme déjà dit, la pression à laquelle la substance est injectée dépend de la vitesse de déplacement et de la force des vérins, par exemple pneumatiques, qui sont également programmés.

L'unité d'injection est alimentée en principe actif par une unité de stockage. En pratique, l'alimentation en liquide de l'unité d'injection est effectuée avec des volumes prédéterminés correspondant au volume injecté dans la tumeur. Le réservoir est séparé de la chambre par un clapet anti-retour, interdisant le retour du liquide dans le réservoir de stockage sous pression du piston. Une fois le liquide froid sous pression propulsé jusqu'au microtube, le microtube connecté à une source électrique, est soumis à un pulse de courant basse tension concomitant au pulse d'eau froide, permettant de chauffer le liquide jusqu'à une température de 400°C. Plus précisément, la source électrique est commandée de manière synchrone avec l'unité d'injection.

La rallonge constitue en réalité, sous forme d'un élément indépendant, le prolongement du tube métallique, partie du tube implantable. Cela signifie donc que la rallonge a une résistivité comprise entre 20 et 100 µΩ.cm. Elle a une longueur comprise entre 2 et 3 m. Son diamètre externe est compris entre 200 µm et 800 µm avantageusement égal à 250 µm, de même que le diamètre interne est compris entre 100 µm et 250 µm, avantageusement égal à 150 µm. De manière générale, plus le diamètre sera élevé et plus grand sera le volume mort, ce qui n'est pas satisfaisant. Comme déjà dit, le tube métallique est réalisé en un matériau du type alliage platine/irridium, titane, acier inoxydable, alliage nickel-titane et plus généralement, tout matériau conducteur électrique apte à supporter une pression jusqu'à 3000 bars et une température de 400°C. La rallonge est connectée par tout moyen connu à l'unité d'injection d'un côté, et l'extrémité proximale du microtube, de l'autre.

Comme déjà dit, l'un des problèmes de l'invention est de fournir une installation grâce à laquelle on puisse injecter du chaud ou du froid en fonction de la nature du traitement. Cet objectif est rempli puisqu'il suffit de déconnecter la source électrique au moment de l'injection de la substance froide. En outre, le régime de pulse, combiné au fait que seule la partie distale du tube implantable est chauffée permet d'enchaîner injection de chaud et de froid sans attente, puisque l'ensemble rallonge et tube implantable, à l'exception de la partie distale, reste froid.

La substance active froide peut revêtir différentes formes, par exemple sous forme d'une suspension de nanocapsules, nanoparticules ou microparticules. On peut ainsi envisager tous types de substances actives, que ce soient celles utilisées en chimiothérapie ou encore en antibiothérapie, de même que les anti-inflammatoires et les produits radioactifs à visée thérapeutique, et ce de façon non limitative.

Dans une forme de réalisation avantageuse, la substance active peut être associée à des nanoparticules magnétiques de ferrite de taille comprise entre 100 et 1000 nanomètres.

Il s'ensuit que lors de l'injection de la substance active à travers le tube, l'énergie communiquée aux nanoparticules magnétiques fait qu'elles se comportent de façon indépendante les unes des autres, leur attraction mutuelle magnétique devenant en effet négligeable par rapport à leur énergie cinétique. En revanche, après injection, c'est-à-dire in situ, l'attraction magnétique favorise le regroupement des nanoparticules sous forme d'amas de tailles d'environ 50 micromètres, dans la zone du tissu à traiter.

Dans le cas d'un principe actif radioactif, ledit principe actif radioactif peut revêtir deux formes différentes :
- soit, il est constitué d'isotopes radioactifs et greffé sur les particules magnétiques ;
- soit, il est inclus dans la particule magnétique et est constitué d'isotopes radioactifs des éléments magnétiques formant les particules magnétiques.

Avantageusement, le produit radioactif peut être émetteur de rayonnement α, β et γ à visée thérapeutique, de préférence de faible énergie, pour obtenir une irradiation la plus locale possible. Il peut être également utile d'associer un émetteur γ d'énergie comprise entre 100 et 150 kiloélectronvolts (Kev) ou émetteur β+ pour visualiser la localisation des nanoparticules à l'aide d'une γ-caméra. Ceci permet en outre de faciliter le calcul de la dose d'irradiation.

Comme déjà dit, on peut utiliser en tant que particules magnétiques des nanoparticules de ferrite.

Dans ce cas, le produit stable donnant le produit radioactif par irradiation par neutrons ou particules chargées est incorporé lors de la fabrication de nanoparticules de ferrite, les composants de la ferrite donnant après irradiation, des produits radioactifs parasites de très courte période, disparaissant donc très vite. De la sorte, seule persiste la radioactivité de l'élément radioactif thérapeutique choisi.

Dans une autre forme de réalisation, on peut associer une substance active à du mercure (Hg) liquide ou en amalgame sous forme de nanoparticules. En effet, lors de l'injection, le mercure liquide prend la forme de micro-gouttes dont l'énergie cinétique est élevée en raison de sa forte densité. In situ, c'est-à-dire au niveau de l'organe, la tension superficielle importante du mercure favorise le regroupement des micro-gouttes en billes plus grosses fixant ainsi la substance active dans l'organe à traiter.

De plus, le mercure possède un isotope radioactif (Hg 197) bien adapté à la thérapie. De la sorte, le principe actif Hg 197 est inclu dans la nanoparticule de mercure. En outre et comme déjà dit, le mercure réalise des amalgames avec la plupart des métaux, ce qui permet donc de fixer d'autres produits radioactifs métalliques sous forme de traces, le mercure restant liquide.

L'invention et les avantages qui en découlent ressortiront bien de l'exemple de réalisation ci-après, à l'appui des figures annexées.
La figure 1 est une représentation schématique de l'installation intégrant le tube implantable de l'invention.
La figure 2 est une représentation du tube implantable de l'invention selon un premier mode de réalisation.
La figure 3 est un agrandissement de la partie distale du tube de la figure 2.
La figure 4 est un second mode de réalisation du tube implantable de l'invention.

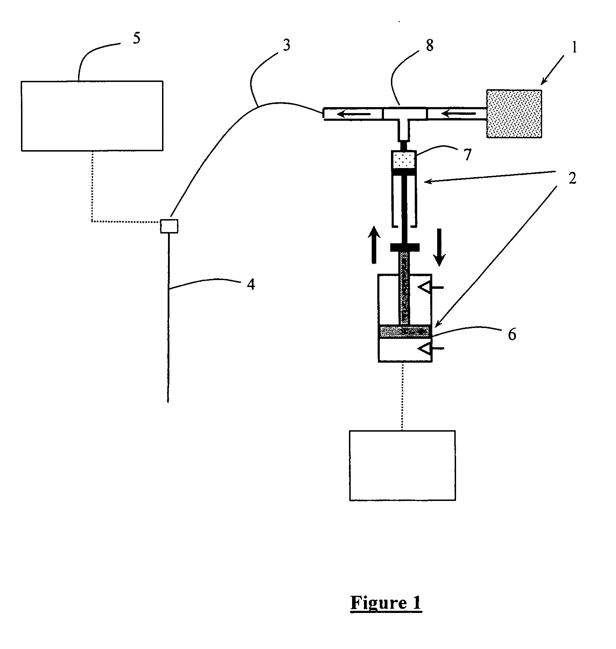

Sur la figure 1, on a représenté schématiquement une installation destinée au traitement par thermoablation de tissus, et en particulier de tumeurs. Plus précisément, cette installation est destinée à délivrer en régime pulsé, directement au niveau de la tumeur, de l'eau sous forme vaporisée à une température d'environ 400°C.

L'installation comprend cinq éléments principaux, respectivement une unité de stockage de liquide à vaporiser (1), une unité d'injection (2), une rallonge (3), le tube implantable de l'invention (4) et un générateur de courant (5) intégrant un système de programmation des pulses, de gestion de capteurs de pression et de température et de mouvement.

L'unité d'injection (2) comprend un vérin pneumatique de grande dimension (6) solidaire d'un vérin hydraulique de petite dimension se déplaçant dans une chambre (7) où le liquide est mis sous pression, débouchant elle-même dans un tube (8) alimenté par l'unité de stockage (1).

La course, la force et la vitesse de déplacement du vérin pneumatique sont programmées en fonction du rythme, du volume et de la pression d'injection souhaitée du liquide caloporteur dans le tube (8).

La rallonge (3) se présente en pratique sous la forme d'un tube d'une taille de l'ordre de 2.5 m, dont le diamètre externe est égal à 250 µm et le diamètre interne à 150 µm. Elle est réalisée en platine/irridium, de résistivité égale à 25 µΩ.cm. Comme il sera vu ensuite, elle constitue le prolongement du tube métallique du tube implantable. La rallonge est connectée, à son extrémité proximale, au tube (8) et à son extrémité distale, au tube implantable (4) par un système type luer-lock, ou HPLC.

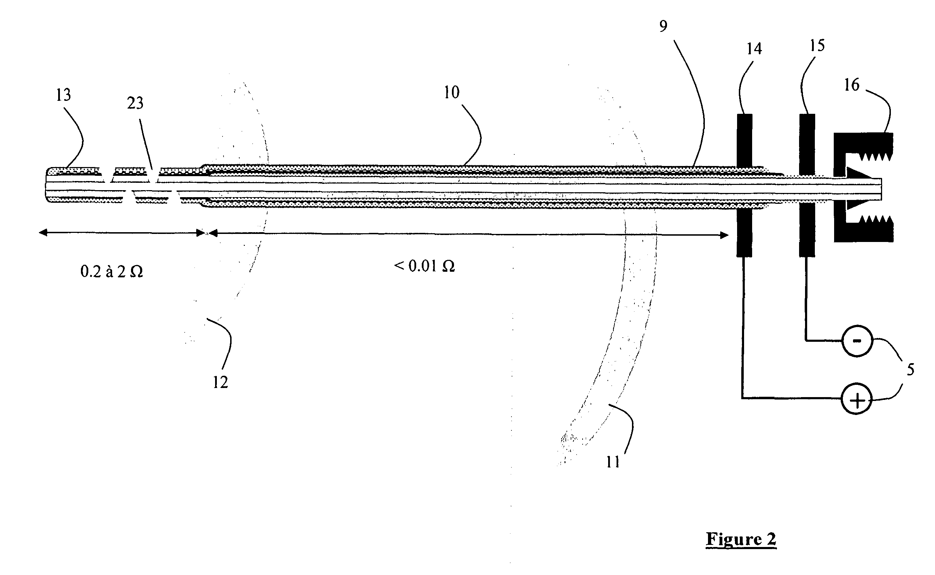

Le microtube (4) est plus particulièrement représenté sur la figure 2. Il est divisé en trois parties, respectivement :
■ une partie proximale (9) correspondant en pratique à la partie non implantée du tube au moment du traitement ;
■ une partie médiane (10) correspondant en pratique à la portion du tube lorsqu'il est implanté, située entre le plan cutané (11) et la zone à traiter (12), et
■ une portion distale (13) située, au moment du traitement, dans la zone à traiter.

Le microtube implantable présente en outre à son extrémité proximale des moyens de connexion (14, 15) aux bornes de la source électrique (5), ainsi qu'un moyen de connexion (16) à l'extrémité distale de la rallonge (3).

Selon une caractéristique essentielle, les parties médiane et proximale du tube présentent une résistance inférieure à 0,01 Ω, tandis que la partie proximale a une résistance comprise entre 0,2 et 2 Ω, les parties médiane et proximale étant reliées électriquement en série à la partie distale. La différence de résistance de ces zones permet de chauffer le liquide exclusivement au niveau de la zone distale du microtube, et non entre la zone à traiter et le plan cutané, évitant ainsi d'échauffer les tissus non concernés par le traitement.

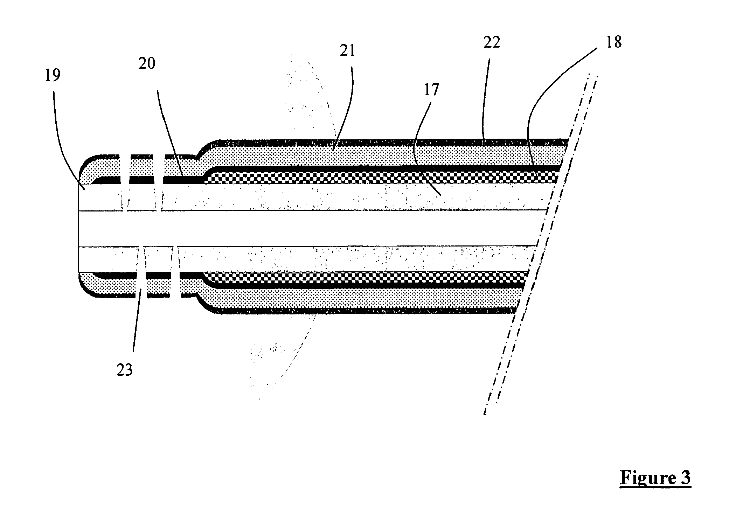

On a représenté sur la figure 3 un agrandissement de la structure du microtube.

Ce tube est constitué du centre vers la périphérie d'un tube proprement dit (17) réalisé en platine/irridium, présentant une longueur variable en fonction de la profondeur de la zone à traiter. En pratique, la taille du tube est inférieure à 50 cm. Le matériau constitutif du tube a une résistivité égale à 25 µΩ.cm et présente un diamètre interne égal à 150 µm et un diamètre externe égal à 250 µm

Dans ces parties médiane et proximale, le tube (17) est recouvert d'un revêtement électrolytique de cuivre (18) d'épaisseur égale à 30 µm en contact au niveau de l'extrémité proximale avec la connexion (15) à la source d'électricité (5) assurant l'arrivée du courant. La partie distale du tube (17), à l'exception de son extrémité distale (19), est recouverte d'une gaine isolante électriquement (20) réalisée en un revêtement sous vide d'oxyde de titane, cette gaine recouvrant le revêtement de cuivre assurant l'arrivée du courant sur les parties médiane et proximale du tube.

Sur la gaine isolante (20), et en contact avec l'extrémité distale (19) du tube (17), le tube implantable de l'invention présente un revêtement électrolytique de cuivre (21) assurant le retour du courant, lequel est en contact avec le second connecteur (14) relié à la source de courant (5). La gaine la plus extérieure est une gaine biocompatible (22) se présentant sous la forme d'un revêtement électrolytique d'or.

En pratique, le liquide froid arrive à l'extrémité proximale du tube sous forme de pulse et est légèrement réchauffé sous l'effet des pulses synchrones de courant. Ce réchauffement reste faible du fait du choix de la résistance, inférieure à 0,01 Ω. Au niveau de la partie distale, le courant circule directement dans la paroi du tube (17) pour repartir au niveau de l'extrémité distale (19) par la gaine (20).

Le tube implantable de l'invention peut présenter deux conformations distinctes.

Dans la première conformation représentée sur la figure 2, celui-ci est obturé à son extrémité libre distale (19) et présente, dans la partie distale, des perforations (23) de taille égale à 70 micromètres.

Ce type de microtube est utilisé pour les traitements au long cours, nécessitant le maintien du microtube dans l'organisme jusqu'à la fin dudit traitement. La mise en place du tube s'effectue au moyen d'une aiguille de ponction latéralement fendue et servant de guide. Une fois que l'ensemble est introduit dans le tissu à traiter, l'aiguille est dégagée du tube, puis retirée.

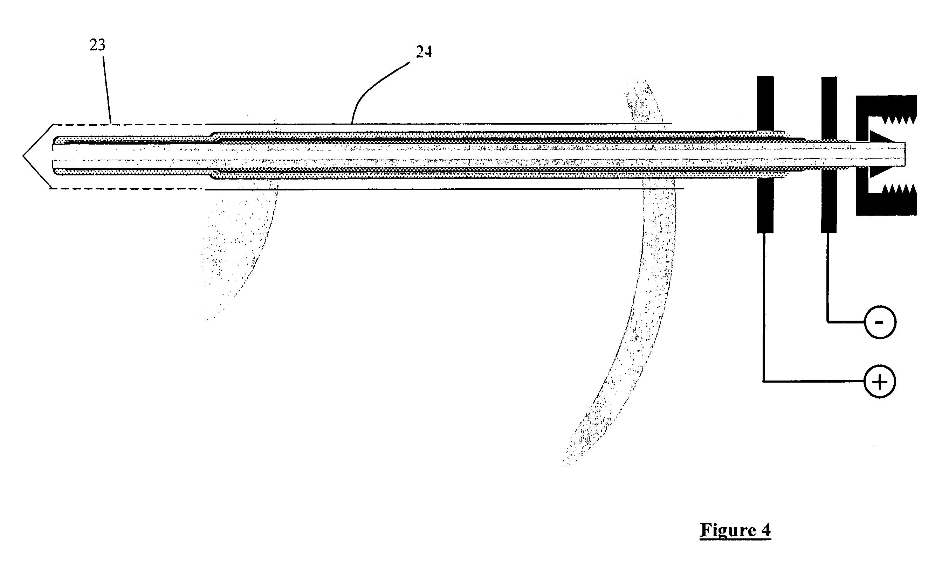

Dans une seconde forme de réalisation, le tube implantable de l'invention est utilisé pour des traitements ponctuels. Dans cette hypothèse, le tube est retiré systématiquement après chaque intervention. Dans ce cas, on met en oeuvre le système représenté sur la figure 4.

En pratique, le microtube est identique à celui représenté sur la figure 2, si ce n'est qu'il présente une extrémité libre distale ouverte, et qu'il est dépourvu de perforations. Les perforations (23) sont en revanche prévues dans l'aiguille de ponction (24) introduite dans le tissu à traiter.

Bien entendu, ces aiguilles de ponction perforées peuvent être également utilisées avec des microtubes eux-mêmes perforés, tels que représentés sur la figure 3.

L'utilisation du système va maintenant être décrite plus en détail.

L'opérateur détermine le volume de substance à injecter en fonction de la taille de la tumeur. De par son expérience, dans le cas de la thermoablation, le Demandeur a constaté qu'il était nécessaire, en général, d'injecter un volume de liquide représentant 5 à 10% du volume de la tumeur à traiter pour obtenir une nécrose satisfaisante (à 400°C). L'opérateur détermine ensuite le volume de chaque injection et en déduit le nombre d'impulsions nécessaires pour parvenir à délivrer le volume total de liquide. Le déclenchement, la course, la force et la vitesse du vérin pneumatique sont alors programmés pour permettre l'injection de N fois le volume de liquide à intervalles réguliers, en pratique compris entre 0,05 et 1 ml, par pulse de durée comprise entre 1 et 2 secondes à une pression de 2 200 bars.

La manipulation débute alors en injectant le premier volume d'eau froide dans la rallonge. En même temps que l'injection, une tension de 6 à 20 Volts est appliquée aux bornes (14) et (15) du tube. La résistance très faible des parties proximale et médiane du tube permet de limiter l'échauffement du fluide, la température étant en pratique de l'ordre de 45°C. Au niveau de la zone distale qui présente une résistance égale à 2 Ω, la température atteint en 4 à 5 secondes, 400°C pour une pression de 2 200 bars. A l'impulsion d'eau froide suivante, l'eau alors vaporisée s'échappe par les perforations du tube ou par son extrémité distale et la vapeur se condense en eau chaude voisine de la température d'ébullition par libération de calories dans la tumeur.

L'invention et les avantages qui en découlent ressortent bien de la description qui précède. On note en particulier l'absence d'unité de chauffage indépendante et l'avantage de pouvoir chauffer un certain volume d'eau directement au niveau de la zone à traiter.

## Revendications

1. Tube implantable (4) apte à chauffer par conduction notamment un fluide caloporteur destiné à être injecté dans tout ou partie d'un tissu humain ou animal, le tube étant muni d'une paroi présentant des parties distale (13) en contact avec la zone à traiter, médiane (10) et proximale (9) et comprenant des moyens de solidarisation directs ou indirects (16) de la partie proximale à un réservoir de fluide, ledit tube étant également muni de deux moyens de connexion (14, 15) aux bornes d'une source électrique (15) assurant l'arrivée et le retour du courant dans la paroi, **caractérisé en ce qu'**il présente une résistance inférieure à 0.01 Ω dans ses parties médiane (10) et proximale (9) et une résistance comprise entre 0.2 et 2 Ω dans sa partie distale (13), les parties médiane (10) et proximale (9) étant reliées électriquement en série à la partie distale (13).

2. Tube selon la revendication 1, **caractérisé en ce que** la paroi présente en section, du centre vers la périphérie :
- un tube métallique (17) dans lequel circule le fluide à injecter,
- à l'exception de la partie distale (13), une gaine (18) assurant l'arrivée du courant, réalisée en un matériau conducteur solidaire d'un moyen de connexion (15) à une des bornes de la source électrique (5),
- à l'exception de l'extrémité libre (19) de la partie distale (13), une gaine (20) isolante électriquement,
- une gaine (21) assurant le retour du courant, réalisée en un matériau conducteur solidaire d'un moyen de connexion (14) à l'autre borne de la source électrique (5).

3. Tube selon l'une des revendications précédentes, **caractérisé en ce que** la paroi est munie d'une gaine extérieure biocompatible (22).

4. Tube selon l'une des revendications 2 ou 3, **caractérisé en ce que** le tube métallique (17) a une résistivité comprise entre 20 et 100 µΩ.cm.

5. Tube selon l'une des revendications 2 à 4, **caractérisé en ce que** le tube métallique (17) présente les caractéristiques suivantes :
- longueur inférieure à 50 cm,
- diamètre externe compris entre 200 µm et 800 µm, avantageusement égal à 250 µm,
- diamètre interne compris entre 100 µm et 250 µm, avantageusement égal à 150 µm.

6. Tube selon la revendication 4, **caractérisé en ce que** le tube métallique (17) est réalisé en un alliage platine/irridium, ou nickel-titane, ou titane ou acier inoxydable.

7. Tube selon la revendication 2, **caractérisé en ce que** la gaine (18, 21) assurant l'arrivée et le retour du courant est constituée d'un revêtement électrolytique de cuivre d'épaisseur comprise entre 20 µm et 50 µm.

8. Tube selon la revendication 2, **caractérisé en ce que** la gaine (20) isolante électriquement est constituée d'un revêtement sous vide d'oxyde de titane d'épaisseur comprise entre 200 nm et 400 nm.

9. Tube selon la revendication 3, **caractérisé en ce que** la gaine (22) extérieure est réalisée en un revêtement électrolytique d'or d'épaisseur d'environ 1 µm.

10. Tube selon l'une des revendications 1 à 9, **caractérisé en ce que** la partie distale (13) est munie de perforations (23) et **en ce que** son extrémité libre est obturée.

11. Tube selon la revendication 10, **caractérisé en ce que** les perforations (23) ont une taille comprise entre 50 µm et 150 µm.

12. Tube selon l'une des revendications 1 à 9, **caractérisé en ce que** l'extrémité libre (19) de la partie distale (13) est munie d'un orifice débouchant.

13. Installation destinée à l'injection notamment de fluide caloporteur en régime pulsé, dans tout ou partie d'un tissu humain ou animal, **caractérisée en ce qu'**elle contient :
- une unité de stockage (1) du fluide;
- une unité d'injection (2) dudit fluide ;
- le tube implantable (4) objet de l'une des revendications 1 à 12 ;
- une rallonge (3) reliant l'extrémité proximale du tube implantable (4) à l'unité d'injection (2).

14. Installation selon la revendication 13, **caractérisée en ce que** l'unité d'injection (2) se présente sous forme d'une chambre (7) contenant la substance à injecter et dans laquelle un vérin hydraulique est piloté par un vérin (6) électrique, pneumatique, piézo-électrique ou mécanique, dont le déclenchement et/ou la course et/ou la force et/ou la vitesse de déplacement sont déterminés en fonction du rythme, du volume et de la pression d'injection souhaitée de la substance dans la rallonge (3) par le vérin hydraulique.

## Claims

1. Implantable tube (4), for heating by conduction in particular a heat transfer fluid for injection into all or part of a human or animal tissue, the tube being provided with a wall having a distal part (13) in contact with the zone to be treated, a median part (10) and a proximal part (9) and comprising means for direct or indirect connection (16) of the proximal part to a fluid reservoir, said tube being also provided with two means (14, 15) for connection to the terminals of an electric power supply (15), for incoming and outgoing current in the wall, **characterized in that** it has a resistance less than 0.01Ω in its median (10) and proximal (9) parts, and a resistance of between 0.2 and 2 Ω in its distal part (13), the median (10) and proximal (9) parts being electrically connected in series to the distal part (13).

2. Tube according to Claim 1, **characterized in that** the wall has in a cross section from the centre to the periphery:
- a metal tube (17) in which the fluid to be injected flows,
- with the exception of the distal part (13), a sheath (18) for the incoming current, made from a conducting material joined to means (15) for connection to one of the terminals of the electric power supply (5),
- with the exception of the free end (19) of the distal part (13), an electrically insulating sheath (20),
- a sheath (21) for the outgoing current, made from a conducting material joined to means (14) for connection to the other terminal of the electric power supply (5).

3. Tube according to one of the preceding claims, **characterized in that** the wall is provided with a biocompatible outer sheath (22).

4. Tube according to either of Claims 2 and 3, **characterized in that** the metal tube (17) has a resistivity of between 20 and 100 µΩ.cm.

5. Tube according to one of Claims 2 to 4, **characterized in that** the metal tube (17) has the following characteristics:
- length shorter than 50 cm,
- outside diameter between 200 µm and 800 µm, advantageously equal to 250 µm,
- inside diameter between 100 µm and 250 µm, advantageously equal to 150 µm.

6. Tube according to Claim 4, **characterized in that** the metal tube (17) is made from a platinum/iridium or nickel-titanium alloy, or from titanium or stainless steel.

7. Tube according to Claim 2, **characterized in that** the sheath (18, 21) for the incoming and outgoing current flow comprises a copper electroplating between 20 µm and 50 µm thick.

8. Tube according to Claim 2, **characterized in that** the electrically insulating sheath (20) comprises a titanium dioxide vacuum coating between 200 nm and 400 nm thick.

9. Tube according to Claim 3, **characterized in that** the outer sheath (22) is made from a gold electroplating about 1 µm thick.

10. Tube according to one of Claims 1 to 9, **characterized in that** the distal part (13) is provided with perforations (23) and **in that** its free end is blocked.

11. Tube according to Claim 10, **characterized in that** the perforations (23) have a size of between 50 µm and 150 µm.

12. Tube according to one of Claims 1 to 9, **characterized in that** the free end (19) of the distal part (13) is provided with an open orifice.

13. Installation for injecting a heat transfer fluid in particular in pulsed conditions, into all or part of a human or animal tissue, **characterized in that** it contains:
- a unit (1) for storing the fluid;
- a unit (2) for injecting the said fluid;
- the implantable tube (4) according to one of Claims 1 to 12;
- an extension (3) connecting the proximal end of the implantable tube (4) to the injection unit (2).

14. Installation according to Claim 13, **characterized in that** the injection unit (2) is in the form of a chamber (7) containing the substance to be injected, and in which a hydraulic cylinder is actuated by an electrical, pneumatic, piezoelectric or mechanical cylinder (6) whereof the actuation and/or stroke and/or force and/or speed of travel are determined according to the desired rate, volume and pressure of injection of the substance in the extension (3) by the hydraulic cylinder.

## Patentansprüche

1. Implantierbares Röhrchen (4) geeignet zur Erwärmung insbesondere eines Wärmeübertragungsfluids durch Leitung, das dazu bestimmt ist, in die Gesamtheit oder einen Teil eines menschlichen bzw. tierischen Gewebes injiziert zu werden, wobei das Röhrchen mit einer Wand versehen ist, die einen distalen Abschnitt (13) in Kontakt mit dem zu behandelnden Bereich, einen mittleren Abschnitt (10) und einen proximalen Abschnitt (9) aufweist und Mittel (16) zum direkten oder indirekten festen Verbinden des proximalen Abschnitts mit einem Fluidvorratsbehälter umfasst, wobei das Röhrchen ebenfalls mit zwei Mitteln (14, 15) zum Anschließen an die Anschlussklemmen einer Stromquelle (15) versehen ist, welche die Stromzuführung und -rückleitung in die bzw. aus der Wand gewährleistet, **dadurch gekennzeichnet, dass** es in seinem mittleren (10) und in seinem proximalen Abschnitt (9) einen Widerstand von unter 0,01 Ω und in seinem distalen Abschnitt (13) einen Widerstand zwischen 0,2 und 2 Ω aufweist, wobei der mittlere (10) und der proximale Abschnitt (9) elektrisch in Reihe mit dem distalen Abschnitt (13) verbunden sind.

2. Röhrchen nach Anspruch 1, **dadurch gekennzeichnet, dass** die Wand im Querschnitt vom Zentrum zur Peripherie hin aufweist:
- einen Metalltubus (17), in welchem das zu injizierende Fluid zirkuliert,
- mit Ausnahme vom distalen Abschnitt (13), eine Umhüllung (18), welche die Stromzuführung gewährleistet und aus einem leitfähigen Material hergestellt ist, die fest mit einem Mittel (15) zum Anschließen an eine der Anschlussklemmen der Stromquelle (5) verbunden ist,
- mit Ausnahme vom freien Ende (19) des distalen Abschnitts (13), eine elektrisch isolierende Umhüllung (20),
- eine Umhüllung (21), welche die Stromrückleitung gewährleistet und aus einem leitfähigen Material hergestellt ist, die fest mit einem Mittel (14) zum Anschließen an die andere Anschlussklemme der Stromquelle (5) verbunden ist.

3. Röhrchen nach einem der vorangehenden Ansprüche, **dadurch gekennzeichnet, dass** die Wand mit einer biologisch verträglichen Außenhülle (22) versehen ist.

4. Röhrchen nach einem der Ansprüche 2 oder 3, **dadurch gekennzeichnet, dass** der Metalltubus (17) einen spezifischen Widerstand von zwischen 20 und 100 µΩ hat.

5. Röhrchen nach einem der Ansprüche 2 bis 4, **dadurch gekennzeichnet, dass** der Metalltubus (17) die nachfolgenden Eigenschaften aufweist:
- Länge geringer als 50 cm,
- Außendurchmesser zwischen 200 µm und 800 µm, bevorzugt gleich 250 µm,
- Innendurchmesser zwischen 100 µm und 250 µm, bevorzugt gleich 150 µm.

6. Röhrchen nach Anspruch 4, **dadurch gekennzeichnet, dass** der Metalltubus (17) aus einer Legierung aus Platin/Iridium oder aus Nickel-Titan oder aus Titan oder aus rostfreiem Stahl hergestellt ist.

7. Röhrchen nach Anspruch 2, **dadurch gekennzeichnet, dass** die die Stromzuführung und -rückleitung gewährleistende Umhüllung (18, 21) aus einer elektrolytischen Kupferbeschichtung mit einer Dicke von zwischen 20 µm und 50 µm besteht.

8. Röhrchen nach Anspruch 2, **dadurch gekennzeichnet, dass** die elektrisch isolierende Umhüllung (20) aus einer Vakuumbeschichtung mit Titanoxid mit einer Dicke zwischen 200 nm und 400 nm besteht.

9. Röhrchen nach Anspruch 3, **dadurch gekennzeichnet, dass** die Außenumhülle (22) aus einer elektrolytischen Goldbeschichtung mit einer Dicke von etwa 1 µm hergestellt ist.

10. Röhrchen nach einem der Ansprüche 1 bis 9, **dadurch gekennzeichnet, dass** der distale Abschnitt (13) mit Perforationen (23) versehen ist und dass es am seinem freien Ende verschlossen ist.

11. Röhrchen nach Anspruch 10, **dadurch gekennzeichnet, dass** die Perforationen (23) eine Größe zwischen 50 µm und 150 µm haben.

12. Röhrchen nach einem der Ansprüche 1 bis 9, **dadurch gekennzeichnet, dass** das freie Ende (19) des distalen Abschnitts (13) mit einer durchgängigen Öffnung versehen ist.

13. Vorrichtung, die zum gepulsten Injizieren insbesondere eines Wärmeübertragungsfluids in die Gesamtheit oder in einen Teil eines menschlichen oder tierischen Gewebes bestimmt ist, **dadurch gekennzeichnet, dass** sie enthält:
- Eine Fluidspeichereinheit (1),
- eine Fluidinjiziereinheit (2),
- das implantierbare Röhrchen (4) nach einem der Ansprüche 1 bis 12,
- eine Verlängerung (3), die das proximale Ende des implantierbaren Röhrchens (4) mit der Injiziereinheit (2) verbindet.

14. Vorrichtung nach Anspruch 13, **dadurch gekennzeichnet, dass** die Injiziereinheit (2) in Form einer Kammer (7) vorliegt, welche die zu injizierende Substanz beinhaltet und in welcher ein Hydraulikzylinder von einem elektrischen, pneumatischen, piezoelektrischen oder mechanischen Zylinder (6) angesteuert wird, bei dem das Auslösen und/oder der Hub und/oder die Kraft und/oder die Verstellgeschwindigkeit in Abhängigkeit von gewünschten Einspritztakt, Einspritzvolumen und Einspritzdruck der Substanz in die Verlängerung (3) mittels des Hydraulikzylinders bestimmt werden.
